# EUROPÄISCHE PATENTANMELDUNG

(11) **EP 1 106 163 A2**
(43) Veröffentlichungstag der Anmeldung: **13.06.2001**
(21) Anmeldenummer: 00126157.7
(22) Anmeldetag: 30.11.2000
(51) Int. Cl.: A61K 6/083

(54) **Dentales Einbettmaterial**

(30) Priorität: 09.12.1999 DE 19959514
(71) Anmelder: Heraeus Kulzer GmbH & Co.KG, 63450 Hanau (DE)
(72) Erfinder: Savic, Novica, 61273 Wehrheim (DE); Erdrich, Albert, Dr., 61231 Bad Nauheim (DE); Stange, Frank, 61250 Usingen (DE); Korthaus, Bettina, 61389 Schmitten (DE)
(74) Vertreter: Kühn, Hans-Christian

(57) **Zusammenfassung**

Es wird unter anderem ein dentales Einbettmaterial vorgestellt, enthaltend:
10 - 30 Gewichts-% Polyethylenglykol-Dimethacrylat,
40 - 55 Gewichts-% Polymethylmethacrylat,
5 - 15 Gewichts-% hochdisperses Siliziumdioxid,
< 1 Gewichts-% Photoinitiatoren, Stabilisatoren,
0 - 10 Gewichts-% Polyethylenglykol und
10 - 30 Gewichts-% mindestens einer Verbindung aus der Gruppe Urethandimethacrylat Bis-GMA, ethoxyliertes Bis-GMA.

## Beschreibung

Die Erfindung betrifft ein transparentes dentales Einbettmaterial, ein Dentalkit, ein Verfahren zur Herstellung einer Prothese, Verwendungen des Einbettmaterials, eine Prothese, eine individuelle Küvette, ein Dentalvorwallmaterial. In der Dentaltechnik sind mehrere traditionelle Verfahren zur Herstellung von Prothesen bekannt.

In DE 195 02 751 A1 wird ein Verfahren zur Herstellung von Kunststoffmodellen für die Zahntechnik offenbart, bei dem ein photohärtbares Material verwendet wird.

GB 1 113 722 A beschreibt eine an der Luft aushärtbare Zusammensetzung auf der Basis von Mono-, Bis-acrylat oder Methacrylat-Monomeren.

GB 916 075 A beschreibt ein Verfahren zur Herstellung eines gegossenen Compositartikels aus polymerisiertem Methylmethacrylatharz.

In EP 0 142 172 A2 wird u. a. eine polymerisierbare Zusammensetzung offenbart, die aus einer polaren organischen Zusammensetzung aus der Gruppe Säuren und deren Salze, einer multifunktionalen vinylvernetzenden Zusammensetzung und einer Solvens-Zusammensetzung besteht.

DE 36 39 067 A1 beschreibt ein Verfahren zur Herstellung eines Plattengebisses, bei dem unter direkter Bildung eines Plattengebisses mit verbesserter Passgenauigkeit innerhalb kurzer Zeit in sachgemäßer Weise photopolymerisierbare Harze mit unterschiedlichen Viskositäten angewandt werden und als Abdruckmaterial ein Teigprodukt von photopolymerisierbaren Harzen mit unterschiedlichen Viskositäten zum Einsatz gelangen.

In DE 299 20 415 U1 ist ein zahntechnischer Distanzlack beschrieben, der ein Bindemittelgehalt von 5 bis 60 % eines Polyvinylalkohols, Farbpigmente und einen leichtflüchtigen, wassermischbaren Alkohol aufweist.

Bei dem traditionellen Verfahren handelt es sich zum einen um die Einbettung einer Wachsaufstellung mit Gips, bei der Gips mit Wasser angemischt und über die in eine Küvette gesetzte Wachsaufstellung gegossen wird. Nach dem Abbinden des Gipses wird das Wachs ausgebrüht und die Gipsflächen mit Alginat isoliert. Der entstehende Hohlraum kann anschließend durch thermisch oder chemisch initiierten Kunststoff im Injektionsverfahren, Gießverfahren oder Stopf-Pressverfahren ausgefüllt und das Material nach verschiedenen Verfahren polymerisiert werden. Nachteilig bei diesem Verfahren ist der relativ hohe Zeitaufwand und die Tatsache, dass keine optische Kontrolle möglich ist, ob die Küvette vollständig ausgegossen ist. Darüber hinaus ist es nachteilig, keine lichthärtenden Materialien verwenden zu können. In nachteiliger Weise kommt es bei diesem Verfahren zum Austrocknen des Gipses mit damit verbundenen Volumenänderungen (Schrumpf), wobei darüber hinaus Restfeuchtigkeit im Gips am Kunststoff Verfärbungen hervorrufen kann und generell eine Isolierung mit Alginat notwendig ist.

Eine weitere Methode ist die Einbettung der Wachsaufstellung in Hydrokolloid, bei der das Hydrokolloid-Material oder auch Agar-Agar durch Erwärmen auf Temperaturen über +50°C verflüssigt und anschließend über die Wachsaufstellung in eine Küvette gegossen wird. Nach Abkühlen und Verfestigung des Materials wird das Wachs entfernt und der entstandene Hohlraum mit einem 2-K-Autopolymerisat im Gießverfahren ausgegossen. Die 2 Komponenten des Autopolymerisats reagieren mit Aushärtung auf die Vermischung und bilden die gewünschte Roh-Prothesenform. Nachteilig bei diesem Verfahren ist die Tatsache, dass es sich hierbei um ein fehlerträchtiges Verfahren handelt, bei dem Spezialküvetten notwendig sind und im allgemeinen eine schlechte Abformgenauigkeit festzustellen ist, wobei keine Polymerisation über 50°C möglich ist. Dieses Verfahren ist nachteiligerweise nur zur Verwendung mit sehr dünnflüssigen Gießkunststoffen geeignet, was zu stärkerem Schrumpf nach der Polymerisation führt.

Schließlich ist auch die Verwendung von Vorwallsilikon bei teilprothetischen Arbeiten wie Komplettierungen bekannt, bei denen Silikon vestibulär an die Wachsaufstellung anmodelliert wird, am entsprechenden Modell aushärtet und so die Zähne in ihrer Stellung fixiert. Durch die halbseitige Öffnung kann im Anschluß daran Gießkunststoff eingegossen und polymerisiert werden. Nachteilig bei diesem Verfahren ist die Tatsache, dass das Silikon aus Basis und Härter angemischt werden muß, wobei häufig fehlerhafte Mischungsverhältnisse zu einer mangelnden Aushärtung führen. Marktübliche, für diesen Zweck verwendete kondensationsvernetzende Silikone sind nicht transparent, jedoch handanmischbar. Geeignete additionsvernetzende Silikone sind zwar transparent aber aufgrund ihrer Klebrigkeit nicht handanmischbar.

Besonders kondensationsvernetzende Silikone unterliegen durch Wasserabgabe einem Schrumpfungsprozeß, der zu Fehlanpassungen in der Prothese führen kann. Darüber hinaus liegt die Abbindezeit der Silikone bis zur Verwendbarkeit im Bereich von bis zu 10 min, wobei darüber hinaus die Zähne im Silikon durch Ankleben mit cyanacrylathaltigen Klebstoffen oder Wachs fixiert werden müssen.

Aus dem vorgenannten ergibt sich das Problem, mit Hilfe eines neuartigen dentalen Einbettmaterials, eines Dentalkits, eines Verfahrens zur Herstellung einer Prothese, verschiedener Verwendungen des Einbettmaterials sowie einer Prothese die oben genannten Nachteile zu beseitigen. Das Problem besteht insbesondere darin, ein dentales Einbettmaterial bereitzustellen, das eine optimale Abform- und Paßgenauigkeit bei gleichzeitig vorteilhaften Werkstoff- und Handlingseigenschaften sicherstellt.

Dieses Problem wird erfindungsgemäß durch ein transparentes dentales Einbettmaterial nach Anspruch 1, ein Dentalkit nach Anspruch 10, ein Verfahren nach Anspruch 15, Verwendungen nach den Ansprüchen 22 bis 23, eine Prothese nach Anspruch 24, eine individuelle Küvette nach Anspruch 25 und ein Dentalvorwallmaterial nach Anspruch 26 gelöst.

Das erfindungsgemäße dentale Einbettmaterial enthält 10 - 30 Gewichts-% PolyethylenglykolDimethacrylat, 40 - 55 Gewichts-% Polymethylmethacrylat, 5 - 15 Gewichts-% hochdisperses Siliziumdioxid, < 1 Gewichts-% Photoinitiatoren, Stabilisatoren, 0 -10 Gewichts-% Polyethylenglykol und 10 - 30 Gewichts-% mindestens einer Verbindung aus der Gruppe Urethandimethacrylat, Bis-GMA, ethoxyliertes Bis-GMA und ist hochtransparent.

Das Einbettmaterial zeigt optimale Abform- und Paßgenauigkeiten beim Erstellen einer individuellen Küvette, wobei unter dem Begriff "individuelle Küvette" ein ausgehärtetes dentales Einbettmaterial, insbesondere ein erfindungsgemäßes dentales Einbettmaterial zu verstehen ist, das über die in Wachs aufgestellte Arbeit inklusive Zähne modelliert worden ist. Der zwischen Gipssockel und individueller Küvette nach Entfernen des Wachses entstehende Hohlraum kann anschließend mit einem handelsüblichen Gießkunststoff ausgegossen werden. Durch die vorhandene hohe Transparenz der individuellen Küvette ist die Verwendung lichtpolymerisierbaren Materials als Gießkunststoff möglich.

Das erfindungsgemäße Einbettmaterial weist eine sehr hohe Abform- und Paßgenauigkeit auf. Darüber hinaus weist das Material zwar eine gewisse Elastizität auf, ist jedoch auf der anderen Seite ausreichend spröde, um ein einfaches Entfernen der individuellen Küvette bzw. des Vorwalls durch mechanisches Zerbrechen des Materials zu ermöglichen.

Bei Vergleich des erfindungsgemäßen Einbettmaterials mit aus dem Stand der Technik bekannten additionsvernetzenden. transparenten Silikonen (siehe DE 40 05 570 A1) ist folgendes festzustellen:

Es handelt sich bei den bekannten transparenten Silikonen um aus Basis und Katalysator bestehende zweikomponentige Systeme, die mit rohstoffpreislich hohen Materialverlusten beim Anmischen und Verarbeiten verbunden sind (u.a. Wechsel des sogenannten statischen Mischers an den Kartuschen etc.), während das erfindungsgemäße Einbettmaterial als lediglich durch Lichtexposition aushärtbares Ein-Komponenten-Material direkt durch Entnahme aus den jeweiligen Packmitteln und Applizierung verwendet wird. Durch die Wasserlöslichkeit im unpolymerisierten Zustand ist eine einfache Hand- und Werkzeugreinigung gewährleistet.

Während Mischungsverhältnis und Umgebungsbedingungen die Eigenschaften der bekannten Silikone nach der Aushärtung beeinflussen, ist eine solche Abhängigkeit nicht feststellbar

Die Verarbeitungszeit ist beim erfindungsgemäßen Einbettmaterial variabel durch entsprechende Licht-Applizierung steuerbar

Additionsvernetzende Silikone enthalten Anteile an Sauerstoff im Material, die eine Inhibitionsschicht an der Trennfläche zum Kunststoff bewirken, und solche Silikone gasen für einen Zeitraum nach Herstellung Wasserstoff aus, was zu Inhomogenitäten im Kunststoff führen kann. Diese Nachteile sind beim erfindungsgemäßen Einbettmaterial nicht vorhanden.

Die bekannten Silikone weisen eine begrenzte Härte (Shore A 65 - 90) bei sehr hoher Flexibilität auf, während das Einbettmaterial bei ausreichender Elastizität eine größere Härte besitzt und folglich leichter beim Ausbettvorgang (Zerbrechen einer individuellen Küvette bzw. eines Vorwalls) zerbrochen werden kann.

Schließlich ist bei den Silikonen ein Anbinden von weiterem Silikon an bestehendes Silikon oder eine Anbindung von Kunststoff an Silikon chemisch nicht möglich, wobei darüber hinaus Zähne mit Klebewachs oder Cyanacrylat-Kleber befestigt werden müssen, während beim Einbettmaterial ein Anbinden von weiteren Acrylaten und Methacrylaten möglich und kein Einkleben von Zähnen zwingend notwendig ist.

Die oben genannten Vorteile sind auch für eine weitere vorteilhafte Ausgestaltung des erfindungsgemäßen Einbettmaterials zutreffend, das 15 - 20 Gewichts-% Polyethylenglykoldimethacrylat, 50 Gewichts-% Polymethylmethacrylat, 10 - 15 Gewichts-% mindestens einer Verbindung aus der Gruppe Urethandimethacrylat, Bis-GMA, ethoxyliertes Bis-GMA sowie 10 - 13 Gewichts-% hochdisperses Siliziumdioxid, 0,4 - 0,6 Gewichts-% Photoinitiatoren, Stabilisatoren sowie 5 - 10 Gewichts-% Polyethylenglykol enthält.

Schließlich haben sich die folgenden Ausgestaltungen in der Praxis als vorteilhaft bewährt:

Das Polyethylenglykoldimethacrylat weist eine Masse > 500 g/mol auf.

Das Polyethylenglykoldimethacrylat ist bei einer Temperatur von ca. T= + 20 °C fest.

Das Polymethylmethacrylat weist eine Molmasse von > 160.000, eine mittlere Korngröße von 80 - 140 pm und einen Benzoylperoxidgehalt < 0,1 Gew.-% auf.

Das Polymethylmethacrylat ist ein Co-Polymer, das mit bis zu 10 Gew.-% Co-Monomeren hergestellt worden ist.

Der Polyethylenglykol ist bei einer Temperatur von ca. T= +20 °C flüssig und weist eine mittlere Molmasse von >= 200 g/mol auf.

Das Urethandimethacrylat weist eine Mindestmolmasse in Höhe von 450 g/mol auf.

In vorteilhafter Weise liegt das Polymethylmethacrylat als Perlpolymerisat vor, da dieses als Füllstoff aufgrund seiner kugelförmigen Ausgestaltung dem Einbettmaterial homogene Eigenschaften verleiht.

Das erfindungsgemäße Dentalkit enthält mindestens ein erfindungsgemäßes Einbettmaterial.

In vorteilhafter Weise weist das Dentalkit ein dentales Isoliermaterial auf, das 10 - 60 Gewichts% Wasser, 30 - 85 Gew.-% C₂-C₄-Alkohol, 2 - 10 Gew.-% Polyvinylalkohol und 0 - 30 Gewichts% Aceton enthält, da auf diese Art und Weise nicht nur die positiven Eigenschaften des erfindungsgemäßen Einbettmaterials sondern auch mittels des Isoliermaterials gute Filmbildungseigenschaften bereitgestellt werden können.

Die nachfolgenden Ausführungen haben sich in der Praxis als vorteilhaft erwiesen:

Das Isoliermaterial enthält 40 - 50 Gew.-% Wasser, 45 - 55 Gew.-% C₂-C₄-Alkohol, 3 - 8 Gew.-% Polyvinylalkohol und 0 - 5 Gew.-% Aceton.

Der C₂-C₄-Alkohol ist Ethanol.

Der Polyvinylalkohol weist eine Molmasse von >60.000 g/mol auf.

Bei dem einen erfindungsgemäßen Verfahren zur Herstellung einer Prothese wird zunächst eine Zahn-Wachsaufstellung mittels eines erfindungsgemäßen Einbettmaterials zum Aufbau einer individuellen Küvette oder eines Vorwalls übermodelliert und durch Einwirkung elektromagnetischer Strahlung, insbesondere mittels sichtbarem Licht und/oder UV-Strahlung, polymerisiert (ausgehärtet). Anschließend wird die Innenseite des polymerisierten Einbettmaterials mit einem Isoliermaterial beschichtet, um daraufhin mit einem Dentalkunststoff ausgegossen zu werden. Dieser wird anschließend insbesondere mittels elektromagnetischer Strahlung ausgehärtet, kann jedoch auch als Autopolymerisat ausgestaltet sein. Anschließend wird die Prothese durch Zerbrechen des Einbettmaterials ausgebettet.

Die folgenden Ausgestaltungen haben sich in vorteilhafterweise Weise in der Praxis bewährt:.

Das dentale Isoliermaterial enthält 10 - 60 Gewichts-% Wasser, 30 - 85 -% C₂-C₄-Alkohol, 2 - 10 Gew.-% Polyvinylalkohol und 0-30 Gewichts-% Aceton enthält, da auf diese Art und Weise nicht nur die positiven Eigenschaften des erfindungsgemäßen Einbettmaterials sondern auch mittels des Isoliermaterials gute Filmbildungseigenschaften bereitgestellt werden können.

Das Isoliermaterial enthält 40 - 50 Gew.-% Wasser, 45 - 55 Gew.-% C₂-C₄-Alkohol, 3 - 8 Gew.-% Polyvinylalkohol und 0 - 5 Gew.-% Aceton.

Der C₂-C₄-Alkohol ist Ethanol.

Der Polyvinylalkohol weist eine Molmasse von >60.000 g/mol auf.

Wie oben geschildert wird in vorteilhafter Weise bei diesem erfindungsgemäßen Verfahren nicht nur ein erfindungsgemäßes Isoliermaterial sondern darüber hinaus ein erfindungsgemäßes Einbettmaterial verwendet, um nicht nur problemlos den ausgehärteten Prothesen kunststoff von der individuellen Küvette bzw. dem Vorwall zu trennen, sondern darüber hinaus ausgezeichnete Abform- und Paßgenauigkeiten zu erhalten.

Die nach diesem Verfahren hergestellten Prothesen weisen aus obigen Gründen ausgezeichnete Abform- und Paßgenauigkeiten auf.

In vorteilhafter Weise wird als Dentalkunststoff ein mittels elektromagnetischer Strahlung, insbesondere mittels sichtbarem Licht und/oder UV-Strahlung, härtbares Dentalmaterial verwendet, da auf diese Art und Weise eine gezielte Aushärtung durch entsprechendes Bestrahlen des Dentalkunststoffs möglich ist.

Dabei ist es von Vorteil, dass vor dem Beschichten Retentionen eingerichtet werden. Unter Retentionen sind im Erfindungskontext Verbindungsstellen und Fixierungen zu verstehen, beispielsweise Kantenfräsungen, Stifte oder Nutverbindungen, die ein Verkanten, Verschieben oder Verrutschen der Verbindung vom Gipssockel zur individuellen Küvette verhindern, somit zur Sicherstellung einer reversiblen Passung dienen.

Auch bei konventionellen Verfahren und/oder Prothesenwerkstoffen zur Herstellung einer Prothese (siehe oben) ist bei der Verwendung des erfindungsgemäßen Einbettmaterials mit ausgezeichneten Abform- und Paßgenauigkeiten zu rechnen.

Auch die das erfindungsgemäße dentale Einbettmaterial enthaltenden individuellen Küvetten und Vorwallmaterialien weisen die obigen vorteilhaften Eigenschaften auf.

Beim erfindungsgemäßen Verfahren zur Herstellung einer Prothese ist kontextbezogen unter der Bezeichnung "Übermodellierung einer Zahn-Wachsaufstellung mittels eines Einbettmaterials" nicht nur die direkte Übermodellierung einer Zahn-Wachsaufstellung sondern auch die Übermodellierung einer zuvor mit einer Schicht Silikon und darüberliegenden Schicht eines Haftsystems, beispielsweise eines cyanacrylathaltigen Klebstoffes, überzogenen Zahn-Wachsaufstellung zu verstehen.

Schließlich ist die Verwendung eines erfindungsgemäßen Einbettmaterials zur Modellherstellung eines transparenten Gipsersatzes vorteilhaft, da somit die Möglichkeit besteht, den später zwischen Modell und polymerisiertem Einbettmaterial eingegossenen Dentalkunststoff (wo sich vorher die Wachsaufstellung befand) auch von der Innenseite des Modells aushärtend zu belichten, wodurch sich eine sehr gleichmäßige Aushärtung ergibt.

Zusammenfassend lassen sich für das erfindungsgemäße Einbettmaterial folgende charakteristische Eigenschaften und Vorteile zusammenfassen:

Optimale Modellierbarkeit, minimierter Polymerisationsschrumpf, hohe Transparenz, ausreichende Elastizität, nicht zu hohe Sprödigkeit, leichte Zerstörbarkeit durch Zerbrechen und Zerschneiden sowie abwaschbare wasserlösliche Dispersionsschichten, toxikologische Unbedenklichkeit.

Das nachfolgende Beispiel dient zur Erläuterung der Erfindung:

Auf einem Gipssockel wird im Standardverfahren die Aufstellung der Arbeit in Wachs mit Zähnen vorgenommen. Der Gipssockel wird mit Retentionen versehen (Kanten, Nute oder Stifte). Über die Wachsaufstellung wird das pastenartige "individuelle Küvettenmaterial" modelliert. An der Rückseite werden 2 bis 3 Eingußöffnungen angelegt. Anschließend wird in einem Lichtpolymerisationsgerät polymerisiert. Das Wachs wird ausgebrüht und die Forminnenseite mittels Isolierflüssigkeit behandelt. Nach dem Abtrocknen können die in dem Küvettenmaterial gefassten Zähne normal konditioniert werden. Anschließend werden Küvettenoberteil und Gipssockel wieder sorgfältig adaptiert und beispielsweise mit Klebewachs fixiert. Danach kann der Gießkunststoff eingegossen und je nach Art des Materials auf verschiedene Weise polymerisiert werden. Die Ausbettung erfolgt über Zerbrechen der individuellen Küvette.

Mit Verwendung von transparentem Silikon:

Weiterhin ist die Kombination eines Trägers (z.B. aus lichthärtendem Methacrylat, beispielsweise obiges Einbettmaterial, oder Thermoplast) mit einer Schicht Silikon denkbar. Hierbei wird das Silikon auf die Wachsaufstellung aufgebracht und die Aushärtung abgewartet. Anschließend wird ein Haftsystem und das Trägermaterial, beispielsweise das lichthärtende Methacrylatsystem aufgebracht. Die weitere Behandlung erfolgt dann wie oben beschrieben.

| Einbettmaterial (Küvettenmaterial): | | |
|---|---|---|
| Urethandimethacrylat Plex 6661 | Röhm | 12,5 Gew.-% |
| Polyethylenglykol-1000-Dimethacrylat | Röhm | 18,2 Gew.-% |
| Polyethylenglykol 200 | Aldrich | 7,8 Gew.-% |
| C-13-Methacrylsäureester | Röhm | 1,9 Gew.-% |
| Aerosil R974 | Degussa | 9,0 Gew.-% |
| Aerosil 380 | Degussa | 2,0 Gew.-% |
| PMMA-Perlpolymerisat M286 | Röhm | 48,0 Gew.-% |
| Lucirin TPO | BASF | 0,6 Gew.-% |
| | | |

| Isoliermaterial: | | |
|---|---|---|
| Polyvinylalkohol 100000 | Fluka | 5,0 Gew.-% |
| Deionisiertes Wasser | Kulzer | 45,0 Gew.-% |
| Ethanol | Brenntag | 50,0 Gew.-% |
| | | |

| Silikone: | | |
|---|---|---|
| Vernetzendes Polyvinylsiloxan | | |
| Memosil CD | HKKG | 100 Gew.-% |

## Patentansprüche

1. Transparentes dentales Einbettmaterial, enthaltend:
| | |
|---|---|
| 10 - 30 | Gewichts-% Polyethylenglykol-Dimethacrylat, |
| 40 - 55 | Gewichts-% Polymethylmethacrylat, |
| 5 - 15 | Gewichts-% hochdisperses Siliziumdioxid, |
| < 1 | Gewichts-% Photoinitiatoren, Stabilisatoren, |
| 0 - 10 | Gewichts-% Polyethylenglykol und |
| 10 - 30 | Gewichts-% mindestens einer Verbindung aus der Gruppe Urethandimethacrylat, Bis-GMA, ethoxyliertes Bis-GMA. |

2. Transparentes dentales Einbettmaterial nach Anspruch 1, enthaltend:
| | |
|---|---|
| 15 - 20 | Gewichts-% Polyethylenglykoldimethacrylat, |
| 50 | Gewichts-% Polymethylmethacrylat, |
| 10 - 15 | Gewichts-% mindestens einer Verbindung aus der Gruppe Urethandimethacrylat, Bis-GMA, ethoxyliertes Bis-GMA, |
| 10 - 13 | Gewichts-% hochdisperses Siliziumdioxid, |
| 0,4 - 0,6 | Gewichts-% Photoinitiatoren, Stabilisatoren, |
| 5 - 10 | Gewichts-% Polyethylenglykol. |

3. Transparentes dentales Einbettmaterial nach einem der Ansprüche 1 bis 2, dadurch gekennzeichnet, dass das Polyethylenglykoldimethacrylat eine Molmasse > 500 g/mol aufweist.

4. Transparentes dentales Einbettmaterial nach einem der Ansprüche 1 bis 3, dadurch gekennzeichnet, dass das Polyethylenglykoldimethacrylat bei einer Temperatur von ca. T= + 20 °C fest ist.

5. Transparentes dentales Einbettmaterial nach einem der Ansprüche 1 bis 4, dadurch gekennzeichnet, dass das Polymethylmethacrylat eine Molmasse von > 160.000, eine mittlere Korngröße von 80 - 140 µm und einen Benzoylperoxidgehalt < 0,1 Gew.-% aufweist.

6. Transparentes dentales Einbettmaterial nach einem der Ansprüche 1 bis 5, dadurch gekennzeichnet, dass das Polymethylmethacrylat ein Co-Polymer ist, das mit bis zu 10 Gew.-% Co-Monomeren hergestellt worden ist.

7. Transparentes dentales Einbettmaterial nach einem der Ansprüche 1 bis 6, dadurch gekennzeichnet, dass der Polyethylenglykol bei einer Temperatur von ca. T= + 20 °C flüssig ist und eine mittlere Molmasse von >= 200 g/mol aufweist.

8. Transparentes dentales Einbettmaterial nach einem der Ansprüche 1 bis 7, dadurch gekennzeichnet, dass das Urethandimethacrylat eine Mindestmolmasse in Höhe von 450 g/mol aufweist.

9. Transparentes dentales Einbettmaterial nach einem der Ansprüche 1 bis 8. dadurch gekennzeichnet, dass das Polymethylmethacrylat als Perlpolymerisat vorliegt.

10. Dentalkit, enthaltend mindestens ein Einbettmaterial nach einem der Ansprüche 1 bis 9.

11. Dentalkit nach Anspruch 10, dadurch gekennzeichnet, dass dieses ein dentales Isoliermaterial aufweist, enthaltend:
| | |
|---|---|
| 10 - 60 | Gewichts-% Wasser, |
| 30 - 85 | Gewichts-% C₂-C₄-Alkohol, |
| 2 - 10 | Gewichts-% Polyvinylalkohol und |
| 0 - 30 | Gewichts-% Aceton. |

12. Dentalkit nach einem der Ansprüche 10 bis 11, dadurch gekennzeichnet, dass dieses ein dentales Isoliermaterial aufweist, enthaltend:
| | |
|---|---|
| 40 - 50 | Gewichts-% Wasser, |
| 45 - 55 | Gewichts-% C₂-C₄-Alkohol, |
| 3 - 8 | Gewichts-% Polyvinylalkohol, |
| 0 - 5 | Gewichts-% Aceton. |

13. Dentalkit nach einem der Ansprüche 10 bis 12, dadurch gekennzeichnet, dass beim den talen Isoliermaterial der C₂-C₄-Alkohol Ethanol ist.

14. Dentalkit nach einem der Ansprüche 10 bis 13, dadurch gekennzeichnet, dass beim den talen Isoliermaterial der Polyvinylalkohol eine Molmasse von > 60.000 g/mol aufweist.

15. Verfahren zur Herstellung einer Prothese mit folgenden Schritten:
a.) Übermodellierung einer Zahn-Wachsaufstellung mittels eines Einbettmaterials nach einem der Ansprüche 1 bis 9 zum Aufbau einer individuellen Küvette oder eines Vorwalls,
b.) Aushärtung des Einbettmaterials mittels elektromagnetischer Strahlung,
c.) Beschichten der Innenseite des polymerisierten Einbettmaterials mit einem dentalen 1soliermaterial,
d.) Ausgießen der individuellen Küvette oder des Vorwalls mit einem Dentalkunststoff,
e.) Ausbetten durch Zerbrechen des Einbettmaterials.

16. Verfahren nach Anspruch 15, dadurch gekennzeichnet, dass das dentale Isoliermaterial enthält:
| | |
|---|---|
| 10 - 60 | Gewichts-% Wasser, |
| 30 - 85 | Gewichts-% C₂-C₄-Alkohol, |
| 2 - 10 | Gewichts-% Polyvinylalkohol und |
| 0 - 30 | Gewichts-% Aceton. |

17. Verfahren nach einem der Ansprüche 15 bis 16, dadurch gekennzeichnet, dass das dentale Isoliermaterial enthält:
| | |
|---|---|
| 40 - 50 | Gewichts-% Wasser, |
| 45 - 55 | Gewichts-% C₂-C₄-Alkohol, |
| 3 - 8 | Gewichts-% Polyvinylalkohol, |
| 0 - 5 | Gewichts-% Aceton. |

18. Verfahren nach einem der Ansprüche 15 bis 17, dadurch gekennzeichnet, dass beim dentalen Isoliermaterial der C₂-C₄-Alkohol Ethanol ist.

19. Verfahren nach einem der Ansprüche 15 bis 18, dadurch gekennzeichnet, dass beim dentalen Isoliermaterial der Polyvinylalkohol eine Molmasse von > 60.000 g/mol aufweist.

20. Verfahren nach einem der Ansprüche 15 bis 19, dadurch gekennzeichnet, dass als Dentalkunststoff ein mittels elektromagnetischer Strahlung härtbares Dentalmaterial verwendet wird.

21. Verfahren nach einem der Ansprüche 15 bis 20, dadurch gekennzeichnet, dass nach der Übermodellierung und vor dem Beschichten Retentionen eingerichtet werden.

22. Verwendung eines lichthärtenden Einbettmaterials nach einem der Ansprüche 1 bis 9 zur Herstellung einer Total- oder Teilprothese.

23. Verwendung eines lichthärtenden Einbettmaterials nach einem der Ansprüche 1 bis 9 als individuelle Küvette oder als Vorwallmaterial.

24. Prothese, dadurch gekennzeichnet, dass diese nach einem Verfahren nach einem der Ansprüche15 bis 21 hergestellt worden ist.

25. Individuelle Küvette, enthaltend ein dentales Einbettmaterial nach einem der Ansprüche 1 bis 9.

26. Dentalvorwallmaterial, enthaltend ein dentales Einbettmaterial nach einem der Ansprüche 1 bis 9.
